# EUROPÄISCHE PATENTANMELDUNG

(11) **EP 3 025 639 A1**
(43) Veröffentlichungstag der Anmeldung: **01.06.2016**
(21) Anmeldenummer: 15190501.5
(22) Anmeldetag: 20.10.2015
(51) Int. Cl.: A61B 5/00, A61B 5/0428, A61B 5/026, A61B 7/00, A61B 8/06

(54) **ELEKTROKARDIOGRAPHIESYSTEM**

(30) Priorität: 26.11.2014 US 201462084582 P
(71) Anmelder: BIOTRONIK SE & Co. KG, 12359 Berlin (DE)
(72) Erfinder: Philipp, Jens, verstorben (DE); Fandrey, Stephan, 8910 Affoltern am Albis (CH); Bartels, Marc, 10115 Berlin (DE)
(74) Vertreter: Galander, Marcus

(57) **Zusammenfassung**

Die Erfindung betrifft ein Elektrokardiographiesystem, das eine Sensoranordnung, die Oberflächenelektroden zur Ableitung elektrischer Signale umfasst oder mit einer solchen verbunden werden kann und eine Signalaufbereitungseinheit mit beispielsweise Filtern und Verstärker aufweist, die mit der Sensoranordnung elektrisch verbunden ist oder verbunden werden kann. Außerdem weist das Elektrokardiographiesystem eine Signalauswerteeinheit auf, die über einen ersten Signaleingang mit der Signalaufbereitungseinheit verbunden ist und ausgebildet ist, ein oder mehrere von der Signalaufbereitungseinheit bereitgestellte, aufbereitete EKG Signale zur Gewinnung eines Elektrokardiogrammsignals zu verarbeiten und das Elektrokardiogrammsignal anzuzeigen oder zur Anzeige auszugeben. Die Signalauswerteeinheit weist zusätzlich einen zweiten Signaleingang zum Empfangen von mittels Körpersensoren oder -monitoren gewonnenen Sekundärsignalen auf und ist ausgebildet, die aufbereiteten EKG-Signale in Abhängigkeit jeweils aktueller Sekundärsignale zu verarbeiten.

## Beschreibung

Die Erfindung betrifft ein Elektrokardiographiesystem mit einer Sensoranordnung, die Oberflächenelektroden zur Ableitung elektrischer Signale umfasst.

Bekannte Elektrokardiographiesysteme dienen der Aufnahme von Kardiogrammen. Dazu werden Oberflächenelektroden auf die Haut eines Patienten aufgebracht und elektrische Potentiale erfasst, die als elektrische Signale abgeleitet und durch beispielsweise Verstärken und Filtern aufbereitet werden. Die aufbereiteten Signale werden dann weiterverarbeitet und beispielsweise einem Arzt in Form eines Elektrokardiogramms zur Kenntnis gebracht.

Das Elektrokardiogramm (EKG) spiegelt die mit der Kontraktion und Expansion von Herzkammern und Vorhöfen einhergehenden elektrischen Potentialveränderungen wieder und liefert daher aufschlussreiche Informationen über den jeweiligen akuten Zustand eines Herzens. Deshalb ist es üblich, Elektrokardiogramme fortwährend auch während anderer Untersuchungen oder Behandlungen aufzunehmen. Neben der Fluoroskopie ist daher das n-Kanal-EKG (n>1) das wichtigste Instrument in einer Elektrophysiologischen Untersuchung (EPU), um kardiologische Arrhythmien zu diagnostizieren, beispielsweise bei Katheterisierungen und Ablationen.

Die Magnetresonanztomographie (MRT steht als Abkürzung sowohl für das bildgebende Verfahren der Magnetresonanztomographie als auch für die hierfür notwendigen Magnetresonanztomographen) ist ein bildgebendes Verfahren, mit dem sich Volumenbilder auch von Weichgewebe in Echtzeit aufnehmen lassen. Dazu wird das zu untersuchende Objekt oder die zu untersuchende Person in einem Magnetresonanztomographen (MRT) starken, wechselnden Magnetfeldern ausgesetzt. Die Magnetresonanztomographie bietet einen räumlich und zeitlich aufgelösten Weichteilgewebekontrast und ermöglicht dadurch ein breites Spektrum diagnostischer Anwendungen für schwer zugängliche Organe bzw. Anatomien wie dem Gehirn, der Wirbelsäule, den Gelenken, den Abdomen, dem kardiovaskulären System etc.

Die an sich wünschenswerte Aufnahme von Elektrokardiogrammen während der Durchführung einer Magnetresonanztomographie in einem MRT ist jedoch nicht so einfach, weil bei der Messung von Elektrokardiogramm (EKG)-Signalen im MRT Signalstörungen auftreten können, verursacht u.a. durch den Magnetohydrodynamischen Effekt (MHD). Dieser Effekt wird hervorgerufen durch das hohe statische Hauptmagnetfeld im Magnetresonanztomographen (MRT).

Eine Kombination beider medizinischer Messverfahren EKG und MRT würde es ermöglichen, eine EPU im MRT durchzuführen hin zu interventionellem bzw. intraoperativem MR (iMR) mit dem Ziel, Ablationen präziser und vor allem nichtionisierend für Patient und Krankenhauspersonal durchzuführen.

Das direkte Zusammenführen von EKG und MRT bereitet aus physikalischen Gründen technische Herausforderungen, insbesondere durch die drei voneinander unabhängigen und demzufolge getrennt adressierbaren Hauptkomponenten des MRT, dem starken homogenen statischen Magnetfeld im Tesla-Bereich (1.), den drei orthogonal zueinander gerichteten magnetischen Gradienten- (2.) und den elektromagnetischen Hochfrequenzfeldern (3.).

Der magnetohydrodynamische Effekt (MHD) hat seine physikalische Ursache in bewegten Ladungsträgern in einem Magnetfeld, die durch die Lorentzkraft in eine bestimmte Richtung gezwungen werden, abhängig von der vektoriellen Bewegungsrichtung der Ladungsträger und der Ausrichtung des Magnetfeldes. Der MHD als pseudo-periodische elektrophysiologische Störgröße im EKG wird hauptsächlich durch den Blutfluss im Aortenbogen während der Systole induziert. Dabei werden bewegte Ladungsträger im Blut durch die Lorentzkraft an die Blutgefäßwand gedrückt und temporär dort lokal akkumuliert. Diese temporäre Ladungsdichte überlagert sich als Potential über das aufzunehmende EKG, insbesondere in dessen QRS-Komplex und kann mit dem EKG auf der Hautoberfläche des Patienten gemessen werden.

Eine Hardwarelösung, um den MHD-Beitrag aus dem EKG zu beseitigen nutzt integrierte, auf Lichtwellenleiter basierende 3-kanalige EKG-Monitore, die sich ausschließlich dazu eignen, eine Überwachung der Herzrate von sedierten bzw. Risikopatienten sicherzustellen bzw. um synchronisierte herzschlagbasierte Bildgebungssteuerung (Gating) zu ermöglichen. Für EPUs wichtige morphologische Analysen sind hiermit wegen der schlechten Signalqualität nicht möglich.

Auch ist ein 3-kanaliger Monitor bekannt, der zwar mit in dem Untersuchungsraum des MRTs betrieben werden darf, jedoch oberhalb einer Feldstärke von 40 mT und somit im Isozentrum des Magneten im MRT nicht eingesetzt werden darf.

In der Literatur gibt es zwei prinzipielle Lösungsansätze zur Filterung des MHDs: mathematische modellbasierte Ansätze und klassische digitale Filter. Mathematische Ansätze setzen an, den Blutfluss in der Aorta zu modellieren, daraus den MHD vorauszusagen, um ihn vom verzerrten EKG abzuziehen. Die Forschungsgruppe um Oster und Tse [Oster J., Llinares R., Tse Z., Schmidt E.J., Clifford G. Realistic MHD Modeling Based on MRI Blood Flow Measurements. The International Society for Magnetic Resonance in Medicine 20th Scientific Meeting & Exhibition, 2012 May, Melbourne, Australia] hat, basierend auf einem menschlichen Torso-Modell, ein eigenes bereits vorhandenes mathematisches Modell des MHDs und des EKGs mit einem zusätzlichen Aorten-Modell verbessert und es mit realen kontrastmittelgestützten MRT-Daten validiert. Dabei wurde mit zwei Regions of Interests (ROIs) sowohl in der auf-, als auch in der absteigenden Aorta der Blutfluss in den MRT-Daten ermittelt und auf die Gesamtaorta übertragen. Aus dem quantifizierten Blutfluss wurden dann die Potentiale des MHDs und des EKGs berechnet.

Die Schule der digitalen Filter verfolgt den Ansatz, ein (adaptives) Filter zu trainieren. Zu dieser Gruppe gehören Adaptive Least Mean Square (LMS) Filtering [Magnetic Resonance in Medicine, Volume 71, Issue 3, pages 1336-1347, March 2014], Adaptive Noise Cancellation (ANC) [Journal of Magnetic Resonance Imaging, 2011 May, 33(5), pages 1184-93], Wiener Filtering [Proc. of EUSIPCO 2012 Conference, August 27-31, Bucharest, Romania], and Template Matching [Medical & Biological Engineering & Computing, July 2008, Volume 46, Issue 7, pp 729-733].

Wenn das Ziel ist, EKG-Signale und vom MHD induzierte Störsignale voneinander sauber zu trennen und für diagnostische Zwecke zu nutzen, sind die bekannten Lösungen aus der Industrie unzureichend. Die 3-Kanal-EKGs, die als Monitore zur Patientenüberwachung und zur synchronisierten Bildgebung von MRTs (Gating) benutzt werden können, sind mit dem expliziten Hinweis der Hersteller versehen, keine für EPUs wichtigen kardiologischen diagnostischen Kenngrößen bzw. morphologische Analysen des Herzens von den Messungen abzuleiten.

Die mathematischen Ansätze in der Wissenschaft haben zwar viel zum theoretischen Verständnis des Einflusses externer und interner (bio)physikalischer Größen auf den MHD beigetragen. Die beschriebenen Modelle bilden aber die Komplexität der hämodynamischen Abläufe aufeinanderfolgender Herzschläge eines Individuums nur unzureichend ab, da unvorhersehbare Ereignisse wie spontane bzw. während einer EPU kontrolliert-induzierte Arrhythmien, z.B. durch gezielte Stimulation des Herzens, die individuelle Anatomie des Patienten, sowie Extrasystolen etc., eine korrekte Echtzeit-Trennung von EKG und MHD verhindern könnten.

Filterbasierte Lösungen aus der Forschung kommen aus praktischer Sicht einer Trennung von EKG und MHD am Nächsten und adaptieren erfolgreiche Ansätze aus anderen technischen Bereichen, wie etwa der Telekommunikation oder der Datenkomprimierung. Das reine Übertragen dieser Technologien erweist sich jedoch als unzureichend, da sie der Depolarisierung der einzelnen Herzzellen nicht gerecht werden.

Zudem haben alle Lösungsansätze bei der Trennung von MHD und EKG gemeinsam, dass weder eine oder mehrere zusätzliche direkte oder indirekte unabhängige, dem Blutfluss entsprechende Messgröße, in Echtzeit rückgekoppelt in einen Filter einbezogenwird.

Der Erfindung liegt die Aufgabe zugrunde, eine Messung eines diagnostischen EKGs im MRT zu ermöglichen, die beispielsweise auch eine Elektrophysiologische Untersuchung (EPU) erlaubt.

Erfindungsgemäß wird diese Aufgabe durch ein Elektrokardiographiesystem gelöst, das eine Sensoranordnung, die Oberflächenelektroden zur Ableitung elektrischer Signale umfasst oder mit einer solchen verbunden werden kann und eine Signalaufbereitungseinheit mit beispielsweise Filtern und Verstärker aufweist, die mit der Sensoranordnung elektrisch verbunden ist oder verbunden werden kann. Außerdem weist das Elektrokardiographiesystem eine Signalauswerteeinheit auf, die über einen ersten Signaleingang mit der Signalaufbereitungseinheit verbunden ist und ausgebildet ist, ein oder mehrere von der Signalaufbereitungseinheit bereitgestellte, aufbereitete EKG Signale zur Gewinnung eines Elektrokardiogrammsignals zu verarbeiten und das Elektrokardiogrammsignal anzuzeigen oder zur Anzeige auszugeben. Die Signalauswerteeinheit weist zusätzlich einen zweiten Signaleingang zum Empfangen von mittels Körpersensoren oder -monitoren gewonnenen Sekundärsignalen auf und ist ausgebildet, die aufbereiteten EKG-Signale in Abhängigkeit jeweils aktueller Sekundärsignale zu verarbeiten.

Vorzugsweise ist die Signalauswerteeinheit ausgebildet, in Abhängigkeit der Sekundärsignale eine Kompensationsfunktion zu bilden oder anzupassen und die Kompensationsfunktion auf die aufbereiteten EKG-Signale anzuwenden.

Mit Hilfe der durch weitere unabhängige Sensorik in Echtzeit gewonnenen und bereitgestellten Sekundärsignale kann ein im MRT verfälschtes EKG korrigiert werden, indem der Einfluss des Magnetohydrodynamischen Effekts (MHD) bestimmt und kompensiert wird. Prinzipiell erhält man für das EKG im MRT ein verfälschtes Messsignal, verursacht durch MHD-induzierte Störsignale: EKG(MRT) = EKG + MHD, in den gemessenen Spannungen ausgedrückt: V_{EKG(MRT)} = V_{EKG} + V_{MHD}.

Gemäß einem ersten Aspekt weist die Signalauswerteeinheit hierzu ein adaptives Filter auf, welches durch Kopplung der EKG-Messung mit initialer Filter/Transferfunktion mittels der Sekundärsignale adaptiert werden kann. Ein solches adaptives Filter lässt sich mit relativ wenig zusätzlichem Hardwareaufwand durch Verwendung des schon bei der Elektrophysiologischen Untersuchung (EPU) vorhandenen intrakardialen EKGs und weiterer Sensorik gut realisieren.

Für ein gesundes regelmäßig schlagendes Herz kann eine Transferfunktion zwischen der Messung im MRT und außerhalb aufgestellt werden. Diese Transferfunktion funktioniert solange keine Unregelmäßigkeiten auftreten, also das Signal vorhersagbar bleibt. Bei einer EP-Prozedur ist das in der Regel nicht der Fall, da Arrhythmien auftreten können, zum einen durch die Prozedur an sich - etwa durch gezielte Stimulation des Herzens -, sowie durch die Tatsache, dass es sich fast immer um ein pathologisch verändertes Herz handelt.

Daher ist es vorteilhaft, eine initiale Filterberechnung durchzuführen, indem die Änderung der EKG-Messung am Patienten beim Einführen in den MRT erfasst wird. Diese initiale Filterberechnung wird dann im MRT ständig durch unabhängige Sensorik und Messungen, die die Sekundärsignale liefern, adaptiert, die nicht durch den MHD beeinflusst werden. Dabei können ein oder mehrere unabhängige Sensoren verwendet werden, um die adaptive Filterfunktion zu trainieren und die EKG-Messung zu verbessern.

Zusätzlich oder alternativ zum ersten Aspekt kann das Elektrokardiographiesystem gemäß einem zweiten Aspekt dazu ausgebildet sein, eine parallele Messung und Korrektur eines n-Kanal-Elektrokardiogramms (EKGs, n>1) in einem Magnetresonanztomographen (MRT) für diagnostische Zwecke - sowie der gleichzeitigen Nutzung des Magnetohydrodynamischen Effekts (MHD) für hämodynamische diagnostische Aussagen - durch gleichzeitiges Scannen der Anatomie des Patienten durch den MRT, ein automatisches Segmentieren der Aorta und Berechnen des MHDs aus dem Blutfluss unter der Berücksichtigung von weiteren physiologischen Echtzeitmessungen mit vom EKG unabhängigen bildgebenden Verfahren durchzuführen.

Beide Aspekte adressieren das störungsfreie Messen von elektrophysiologischen Signalen im Umfeld von starken homogenen statischen Magnetfeldern (1), die durch den MHD verfälscht werden. Die Signalauswerteeinheit bereinigt ein in der Umgebung von starken Magnetfeldern aufgezeichnetes n-kanaliges Elektrokardiogramm (EKG, n>1), welches durch induzierte magnetohydrodynamische Störsignale verzerrt wurde, mit dem Ziel, diagnostische Aussagen sowohl über die Hämodynamik als auch über die Elektrophysiologie des Herzens in einer Umgebung starker statischer Magnetfelder treffen zu können.

Vorzugsweise umfasst die Sensoranordnung von den Oberflächenelektroden verschiedene Sekundärsensoren zum Erfassen einer weiteren physikalischen Größe. Die Sekundärsensoren liefern eine oder mehrere Sekundärsignale. Die Signalauswerteeinheit weist (gemäß dem vorgenannten ersten Aspekt) ein adaptives Filter auf, dessen Übertragungsfunktion von dem Sekundärsignal oder den Sekundärsignalen beeinflusst und/oder gesteuert ist. Das adaptive Filter erlaubt eine Messung eines Oberflächen-EKG Signals ohne MHD-induzierte Störsignale. Dabei wird die Adaption des Filters durch eine Auswertung eines oder mehrerer unabhängiger Sensoren erreicht.

Das adaptive Filter weist vorzugsweise eine Übertragungsfunktion auf, die eine Übertragungsfunktion zwischen außerhalb eines MRT aufgenommener EKG Signale und innerhalb der Magnetfelder aufgenommener EKG-Signale kompensiert. Die Übertragungsfunktion des adaptiven Filters ist also zu der durch die Einwirkung des MRT auf das EKG (oder genauer: die im MRT abgeleiteten EKG-Signale) bewirkten Übertragungsfunktion invers.

Die Signalauswerteeinheit ist vorzugsweise ausgebildet, eine initiale Übertragungsfunktion für das adaptive Filter zu bilden, indem sie die Änderung der EKG-Messung am Patienten beim Einführen in den MRT erfasst.

Gemäß einer Ausführungsvariante kann der Sekundärsensor zur Aufnahme eines intrakardialen EKGs ausgebildet sein und ein intrakardiales EKG als Sekundärsignal liefern. Ein intrakardiales EKG kann für die adaptive Filterberechnung geeignete Werte liefern. Ein intrakardiales EKG kann über zusätzliche Diagnostik-Katheter als Sekundärsensoren aufgenommen werden, die bei einer EP-Prozedur in der Regel immer verwendet werden. Aufgrund des kleinen Vektors zwischen den Elektroden bei der Signalableitung eines intrakardialen EKGs hat der MHD bei der Messung im MRT kaum einen Einfluss. Da das Oberflächen-EKG und das intrakardiale EKG zusammenhängen, kann es als vom MHD unabhängiger Messwert zur adaptiven Filterberechnung verwendet werden. Änderungen im Herzrhythmus sind im intrakardialen EKG erkennbar und lassen sich so zur Berechnung heranziehen.

Alternativ oder zusätzlich kann der Sekundärsensor auch ein akustischer Sensor sein, der ausgebildet ist, Herztöne aufzunehmen. Diese werden durch den MHD nicht beeinflusst und können daher zur Filterberechnung und -adaption herangezogen werden.

Alternativ oder zusätzlich kann der Sekundärsensor auch ein Pulsoximeter sein, welches ausgebildet ist, einen Puls zu messen. Auch der Puls wird durch den MHD nicht beeinflusst und kann daher zur Filterberechnung und -adaption herangezogen werden.

Akustischer Sensor und Pulsoximeter sind somit Beispiele für Sekundärsensoren als weitere vom MHD unabhängige Sensoren, die Sekundärsignale liefern können, die in das mathematische Modell vom adaptiven Filter einfließen können.

Gemäß dem zweiten Aspekt ist der zweite Signaleingang vorzugsweise mit einer Einrichtung zur Echtzeitblutflussmessung verbunden, wobei die Sekundärsignale einen Blutfluss in Echtzeit repräsentieren. Die Signalauswerteeinheit ist ausgebildet, aus den Sekundärsignalen mindestens ein Kompensationssignal zu bestimmen, das den Einfluss des Magnetohydrodynamischen Effekts (MHD) widerspiegelt, und dieses Kompensationssignal von einem oder mehreren aufbereiteten EKG Signalen zu subtrahieren oder anderweitig kompensatorisch zu verrechnen.

Auf diese Weise können EKG und vom MHD in der Umgebung starker Magnetfelder induzierte Störsignale getrennt werden, um beide elektrophysiologische Signale anschließend für diagnostische Zwecke nutzen zu können. Dazu wird der dem MHD entsprechende Blutfluss mittels unabhängiger medizinischer Bildgebung in Echtzeit beispielsweise im MRT erfasst und von der Signalauswerteeinheit quantifiziert und als rückgekoppelte Größe einem Filter bzw. einer Filterkaskade zugeführt.

Die Einrichtung zur Echtzeitblutflussmessung kann beispielsweise ein MRT sein, dass an sich bekannte DICOM-Daten liefert (DICOM: Digital Imaging and Communications in Medicine). Die DICOM-Daten stellen dann ein Sekundärsignal im Sinne dieser Beschreibung dar.

Zusätzlich oder alternativ kann die Einrichtung zur Echtzeitblutflussmessung auch eine Ultraschallmesseinrichtung sein, die eine unabhängige nicht-ionisierende UltraschallMessung durchführt.

Vorzugsweise ist die Signalauswerteeinheit ausgebildet, vom MRT stammende DICOM-Daten als Sekundärsignal auszuwerten, indem sie die Aorta automatisch segmentiert und den MHD aus dem Blutfluss in der Aorta berechnet.

Das erfindungsgemäße Elektrokardiographiesystem erlaubt die parallele Messung und Korrektur eines n-Kanal-Elektrokardiogramms (EKGs, n>1) in einem Magnetresonanztomographen (MRT) für diagnostische Zwecke - sowie der gleichzeitigen Nutzung des Magnetohydrodynamischen Effekts (MHD) für hämodynamische diagnostische Aussagen - durch gleichzeitiges Scannen der Anatomie des Patienten durch den MRT, die automatische Segmentierung der Aorta und die Berechnung des MHDs aus dem Blutfluss unter der Berücksichtigung von weiteren physiologischen Echtzeitmessungen mit vom EKG unabhängigen bildgebenden Verfahren.

Ein Vorteil des hiermit vorgeschlagenen Elektrokardiographiesystems ergibt sich aus dem Hinzufügen einer oder mehrerer unabhängiger dem Blutfluss entsprechender Messgrößen, die in Echtzeit einem Filter bzw. einer Filterkaskade rückgekoppelt hinzugeführt werden, um das n-kanalige EKG (n>1) von den vom MHD induzierten Störsignalen zu trennen, um damit diagnostische Aussagen treffen zu können. Das Einbinden unabhängiger bildgebender Verfahren ist besonders, weil das Herausrechnen des MHD aus dem verfälschten EKG durch ihre nicht-stationäre Quellenverwandtschaft theoretisch unmöglich ist, und in der Praxis nur als Annäherung realisiert werden kann.

Die Erfindung soll nun anhand von Ausführungsbeispielen mit Bezug auf die Figuren näher erläutert werden. Von den Figuren zeigt:
- Fig. 1:: Einen Überblick über ein erfindungsgemäßes Elektrokardiographiesystem;
- Fig. 2:: ein Diagramm zur Erläuterung der Funktionsweise einer Signalauswerteeinheit gemäß eines ersten Aspektes der Erfindung;
- Fig. 3:: eine erste Variante eines Elektrokardiographiesystems gemäß eines zweiten Aspekts; und
- Fig. 4:: eine zweite Variante eines Elektrokardiographiesystems gemäß des zweiten Aspekts.

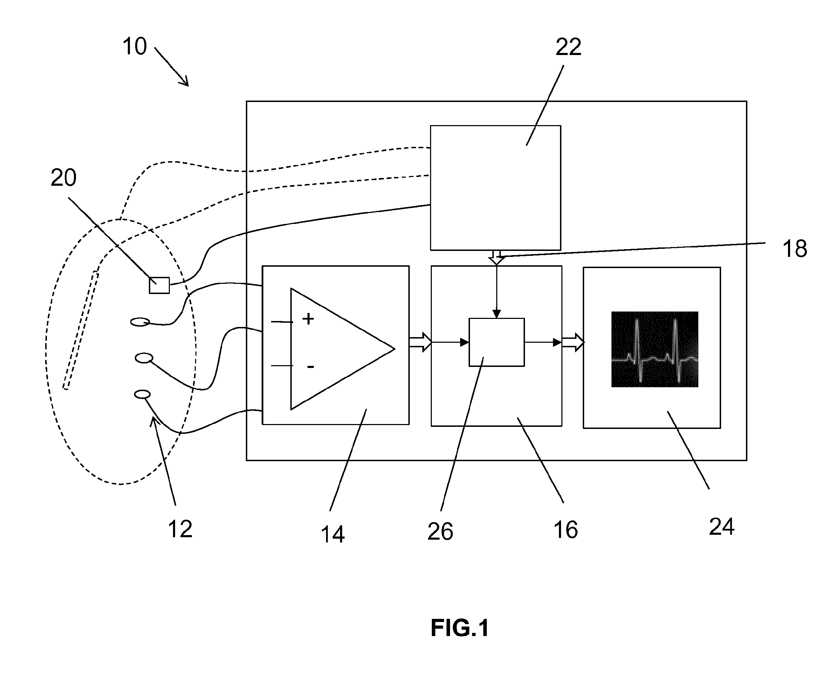

In Fig. 1 sind schematisch die wichtigsten Komponenten eines erfindungsgemäßen Elektrokardiographiesystems 10 dargestellt. Wie bei einem herkömmlichen Elektrokardiographiesystem ist eine Sensoranordnung 12 von Oberflächensensoren zur Ableitung elektrischer Signale vorgesehen. Diese Sensoranordnung ist mit einer Signalaufbereitungseinheit 14 verbunden, die ausgebildet ist, die abgeleiteten elektrischen Signale zu verstärken und zu filtern. Die so aufbereiteten elektrischen Signale werden einer Signalauswerteeinheit 16 zugeführt, die die abgeleiteten und aufbereiteten elektrischen Signale unter Berücksichtigung von Sekundärsignalen (angedeutet durch den Pfeil 18) auswertet.

Die Sekundärsignale 18 stammen von einem Sekundärsensor 20 oder aber auch von mehreren unterschiedlichen Sekundärsensoren. Eine Sekundärsignalaufbereitungseinheit 22 bereitet die vom Sekundärsensor 20 gelieferten Signale auf und gibt entsprechende Sekundärsignale 18 an die Signalauswerteeinheit 16 ab.

Die Signalauswerteeinheit 16 ermittelt aus den aufbereiteten abgeleiteten Signalen der Sensoranordnung 12 und den Sekundärsignalen 18 ein vom Einfluss des magnetohydrodynamischen Effekts (MHD) weitestgehend bereinigtes Elektrokardiogramm (EKG), das auf einer Anzeige oder einem Drucker 24 zur Anzeige gebracht werden kann.

Gemäß einem ersten Aspekt weist die Signalauswerteeinheit 16 ein adaptives Filter 26 auf, das durch die Sekundärsignale 18 gesteuert oder zumindest beeinflusst, beispielsweise adaptiert, ist.

Das adaptive Filter 26 kann eine initiale Übertragungsfunktion besitzen, die die Signalauswerteeinheit 16 aus der Änderung der abgeleiteten elektrischen Signale beim Einführen eines Patienten in einen Magnetresonanztomographen (MRT) bestimmt hat. Vor dem Einführen des Patienten in den MRT sind die abgeleiteten elektrischen Signale noch nicht durch das MRT und insbesondere durch den MHD beeinflusst, während nach Einführen des Patienten in den MRT die abgeleiteten elektrischen Signale durch den MHD beeinflusst sind. Durch Vergleichen der vor dem Einführen des Patienten in den MRT abgeleiteten elektrischen Signale mit den im MRT abgeleiteten elektrischen Signalen lässt sich der Einfluss des MHD bestimmen und daraus eine Übertragungsfunktion ableiten, die Veränderungen der abgeleiteten elektrischen Signale vor und nach Einführen in den MRT beschreibt. Eine hierzu inverse Übertragungsfunktion kann den Einfluss des MHD kompensieren und somit eine initiale Übertragungsfunktion des adaptiven Filters 26 bilden.

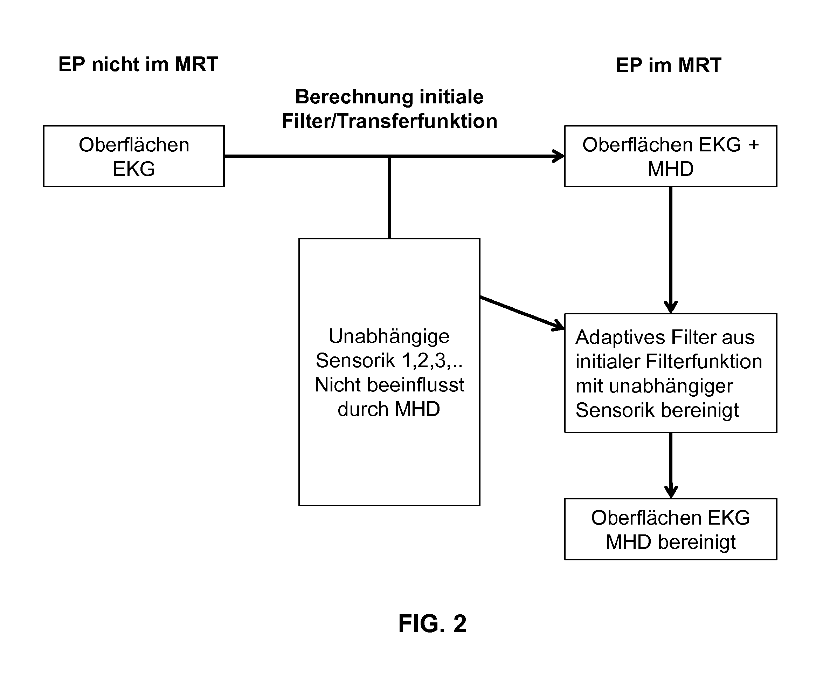

Diese initiale Übertragungsfunktion des adaptiven Filters 26 kann während beispielsweise einer elektrophysiologischen Untersuchung (EPU) eines Patienten im MRT fortwährend und in Echtzeit mit Hilfe des Sekundärsignals oder der Sekundärsignale korrigiert werden, so wie dies in Fig. 2 dargestellt ist.

Ein geeignetes Sekundärsignal ist beispielsweise ein intrakardiales Elektrokardiogramm, das im Unterschied zu einem Oberflächen-Elektrokardiogramm wegen des kleinen Vektors zwischen den intrakardialen Elektroden bei der Signalableitung kaum vom magnetohydrodynamischen Effekt (MHD) beeinflusst ist. Da das Oberflächen-Elektrokardiogramm und das intrakardiale Elektrokardiogramm zusammenhängen, kann das intrakardiale Elektrokardiogramm als vom magnetohydrodynamischen Effekt weitgehend unabhängiges Sekundärsignal zur adaptiven Filterberechnung verwendet werden.

Falls das Sekundärsignal ein intrakardiales Elektrokardiogramm ist, kann der Sekundärsensor 20 beispielsweise ein zusätzlicher Diagnostik-Katheter sein, der bei Elektrophysiologie-Prozeduren in der Regel ohnehin verwendet wird.

Der Sekundärsensor kann aber auch beispielsweise ein akustischer Sensor zur Aufnahme von Herztönen oder ein Pulsoximeter zum Messen des Pulses sein. Beide Sensoren sind deshalb geeignete Sekundärsensoren, weil sie vom MHD unabhängige Sekundärsignale liefern.

Vorzugsweise sind mehrere Sekundärsensoren 20 vorgesehen (angedeutet durch die gestrichelte Darstellung in Fig. 1), die sich in ihrer Funktionsweise unterscheiden, beispielsweise eine Kombination aus einer Sonde zur Aufnahme eines intrakardialen Elektrokardiogramms und einem akustischem Sensor.

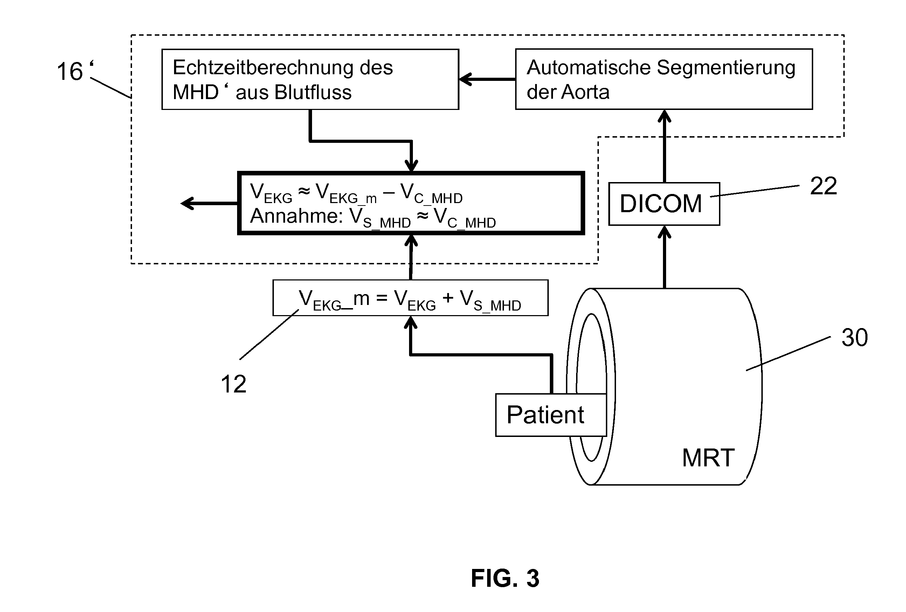

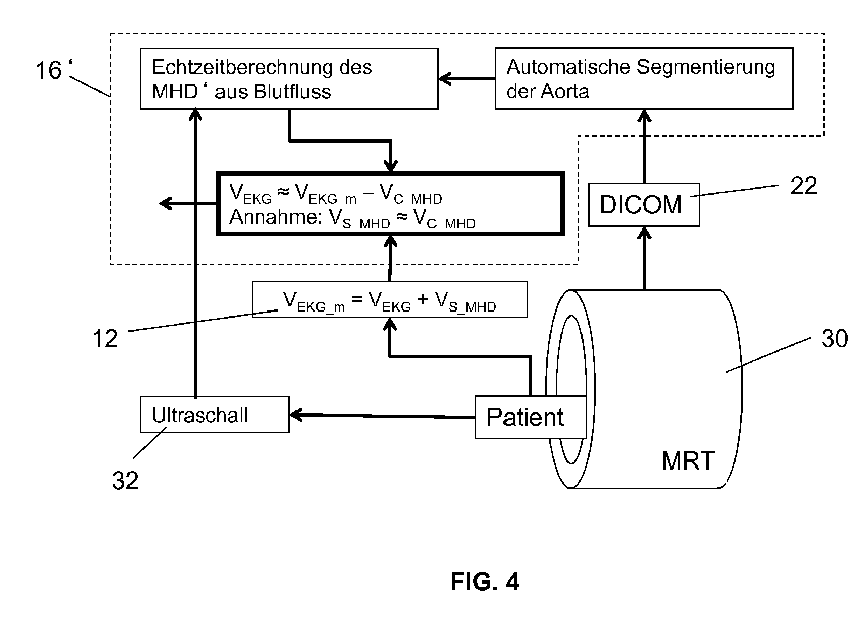

Die Figuren 3 und 4 illustrieren einen zweiten Aspekt, gemäß dem das Sekundärsignal mittels des Magnetresonanztomographen selbst gewonnen wird, indem aus den vom MRT gelieferten DICOM-Daten der jeweils aktuelle Blutfluss in der Aorta bestimmt wird. In diesem Fall ist die Signalauswerteeinheit 16 dazu ausgebildet, aus den DICOM-Daten die Aorta automatisch zu segmentieren und den Blutfluss in der Aorta in Echtzeit zu bestimmen. Aus dem Blutfluss in der Aorta kann der magnetohydrodynamische Effekt (MHD) bestimmt werden. Damit ist es möglich, den Einfluss des magnetohydrodynamischen Effekts auf die abgeleiteten und aufbereiteten Signale zu bestimmen und diesen Einfluss von den abgeleiteten und aufbereitenden elektrischen Signalen abzuziehen, so dass sich ein vom magnetohydrodynamischen Effekt weitestgehend bereinigtes Elektrokardiogramm ergibt. Fig. 3 zeigt hierzu beispielhaft einen Magnetresonanztomographen (MRT) 30 als Sekundärsensor.

Ausgehend von einem im MRT befindlichen Patienten, bei dem die Oberflächenelektroden zur Ableitung eines n-Kanal-EKG (n>1) bereits angelegt wurden, wird eine 4D MRT Bildsequenz (z.B. Phasenkontrast) vom Thorax aufgenommen. Die Verabreichung eines Kontrastmittels ist nicht vorgesehen, aber auch nicht ausgeschlossen. Aus den daraus resultierenden DICOM-Daten wird die Aorta als Grundlage für die Echtzeitblutflussmessung automatisch erkannt und segmentiert. Das im MRT gemessene EKG (EKG_m oder V_{EKG_m}) ist das durch die induzierten Störsignale des MHD (S_MHD oder V_{S_MHD}) additiv verfälschte EKG des Patienten (EKG oder V_{EKG}). Das durch die Echtzeitblutflussmessung berechnete Störsignal des MHDs (C_MHD oder V_{C_MHD}) kann vom verfälschten gemessenen EKG (EKG_m oder V_{EKG_m}) abgezogen werden, unter der Annahme, dass S_MHD oder als Potential ausgedrückt V_{S_MHD} und C_MHD oder als Potential ausgedrückt V_{C_MHD} ähnlich sind.

Die in Fig. 4 dargestellte Ausführungsvariante unterscheidet sich von der in Fig. 3 dargestellten Ausführungsvariante dadurch, dass zusätzlich eine Ultraschalleinrichtung 32 als weiterer Sekundärsensor vorgesehen ist, die dazu ausgebildet ist, den Blutfluss durch Ultraschallmessung zu bestimmen. Diese Bestimmung des Blutflusses per Ultraschall ist durch das MRT nicht beeinflusst und liefert somit einen weiteren Messwert als Sekundärsignal zur Berechnung des Einflusses des MHD durch die Signalauswerteeinheit 16. Demgemäß kann der Einfluss des MHD mit einem Elektrokardiographiesystem gemäß Fig. 4 tendenziell noch genauer bestimmt werden als mit dem Elektrokardiographiesystem gemäß Fig. 3.

Die beiden hier beschriebenen Aspekte - zum Einen Nutzen eines adaptiven Filters zur Kompensation des magnetohydrodynamischen Effektes und zum Anderen Bestimmen des Einflusses des magnetohydrodynamischen Effektes mit Hilfe von Echtzeit-Blutflussmessungen im MRT - lassen sich auch miteinander kombinieren, um auf diese Weise ein Elektrokardiogramm zu finden, das möglichst wenig durch den magnetohydrodynamischen Effekt beeinflusst ist.

### Bezugszeichenliste

- 10: Elektrokardiographiesystem
- 12: Sensoranordnung
- 14: Signalaufbereitungseinheit
- 16: Signalauswerteeinheit
- 18: Sekundärsignal
- 20: Sekundärsensor
- 22: Sekundärsignalaufbereitungseinheit
- 24: Anzeige-/Drucker
- 26: adaptives Filter
- 30: Magnetresonanztomograph (MRT)
- 32: Ultraschalleinrichtung

## Patentansprüche

1. Elektrokardiographiesystem (10) mit einer Sensoranordnung (12), die Oberflächenelektroden zur Ableitung elektrischer Signale umfasst, sowie eine Signalaufbereitungseinheit (14), die mit der Sensoranordnung (12) elektrisch verbunden ist, und eine Signalauswerteeinheit (16), die über einen ersten Signaleingang mit der Signalaufbereitungseinheit (14) verbunden ist und ausgebildet ist, ein oder mehrere von der Signalaufbereitungseinheit (14) bereitgestellte, aufbereitete EKG Signale zur Gewinnung eines Elektrokardiogrammsignals zu verarbeiten und das Elektrokardiogrammsignal anzuzeigen oder zur Anzeige auszugeben,
**dadurch gekennzeichnet, dass** die Signalauswerteeinheit (16) einen zweiten Signaleingang zum Empfangen von mittels Körpersensoren oder -monitoren als Sekundärsensoren (20) gewonnenen Sekundärsignalen (18) aufweist und ausgebildet ist, die aufbereiteten EKG-Signale in Abhängigkeit jeweils aktueller Sekundärsignale (18) zu verarbeiten.

2. Elektrokardiographiesystem (10) nach Anspruch 1, **dadurch gekennzeichnet, dass** die Signalauswerteeinheit (16) ausgebildet ist, in Abhängigkeit der Sekundärsignale (18) eine Kompensationsfunktion zu bilden oder anzupassen und die Kompensationsfunktion auf die aufbereiteten EKG-Signale anzuwenden.

3. Elektrokardiographiesystem (10) nach Anspruch 1 oder 2, **dadurch gekennzeichnet, dass** die Sensoranordnung (12) wenigstens einen von den Oberflächenelektroden verschiedenen Sekundärsensoren (20) zum Erfassen einer weiteren physikalischen Größe umfasst, der eine oder mehrere Sekundärsignale (18) liefert und dass die Signalauswerteeinheit (16) ein adaptives Filter aufweist, dessen Übertragungsfunktion von dem Sekundärsignal (18) oder den Sekundärsignalen beeinflusst und/oder gesteuert ist.

4. Elektrokardiographiesystem (10) nach Anspruch 3, **dadurch gekennzeichnet, dass** das adaptive Filter eine Übertragungsfunktion aufweist, die eine Übertragungsfunktion zwischen außerhalb eines MRT (30) aufgenommener EKG Signale und innerhalb der Magnetfelder aufgenommener EKG-Signale kompensiert.

5. Elektrokardiographiesystem (10) nach Anspruch 3 oder 4, **dadurch gekennzeichnet, dass** die Signalauswerteeinheit (16) ausgebildet ist, eine initiale Übertragungsfunktion für das adaptive Filter zu bilden, indem sie die Änderung der EKG-Messung am Patienten beim Einführen in den MRT (30) erfasst.

6. Elektrokardiographiesystem (10) nach wenigstens einem der Ansprüche 3 bis 5, **dadurch gekennzeichnet, dass** der Sekundärsensor (20) zur Aufnahme eines intrakardialen EKGs ausgebildet ist und das Sekundärsignal (18) ein intrakardiales EKG ist.

7. Elektrokardiographiesystem (10) nach wenigstens einem der Ansprüche 3 bis 6, **dadurch gekennzeichnet, dass** der Sekundärsensor (18) ein akustischer Sensor ist, der ausgebildet ist, Herztöne aufzunehmen.

8. Elektrokardiographiesystem (10) nach wenigstens einem der Ansprüche 3 bis 7, **dadurch gekennzeichnet, dass** der Sekundärsensor (20) ein Pulsoximeter ist, welches ausgebildet ist, einen Puls zu messen.

9. Elektrokardiographiesystem (10) nach Anspruch 1 oder 2, **dadurch gekennzeichnet, dass** der zweite Signaleingang mit einer Einrichtung zur Echtzeitblutflussmessung verbunden ist und die Sekundärsignale (18) einen Blutfluss in Echtzeit repräsentieren, wobei die Signalauswerteeinheit (16) ausgebildet ist, aus den Sekundärsignalen (18) mindestens ein Kompensationssignal zu bestimmen, das den Einfluss des Magnetohydrodynamischen Effekts (MHD) widerspiegelt, und dieses Kompensationssignal von einem oder mehreren aufbereiteten EKG Signalen zu subtrahieren.

10. Elektrokardiographiesystem (10) nach Anspruch 9, **dadurch gekennzeichnet, dass** die Einrichtung zur Echtzeitblutflussmessung ein MRT (30) ist.

11. Elektrokardiographiesystem (10) nach Anspruch 10, **dadurch gekennzeichnet, dass** die Einrichtung zur Echtzeitblutflussmessung eine Ultraschallmesseinrichtung (32) ist.

12. Elektrokardiographiesystem (10) nach wenigstens einem der Ansprüche 9 bis 11, **dadurch gekennzeichnet, dass** die Signalauswerteeinheit (16) ausgebildet ist, vom MRT (30) stammende Daten als Sekundärsignal (18) auszuwerten, indem sie die Aorta automatisch segmentiert und den MHD aus dem Blutfluss in der Aorta berechnet.
